# EUROPÄISCHE PATENTANMELDUNG

(11) **EP 0 663 589 A1**
(43) Veröffentlichungstag der Anmeldung: **19.07.1995**
(21) Anmeldenummer: 94890005.5
(22) Anmeldetag: 13.01.1994
(51) Int. Cl.: G01N 1/10, G01N 33/04

(54) **Verfahren und Vorrichtung zum Identifizieren von aus zugelieferten Flüssigkeitsmengen gezogenen und in Probengefässe eingefüllten Proben, insbesondere Milchproben**

(71) Anmelder: EBNER ELECTRONIC GESELLSCHAFT m.b.H., A-4810 Gmunden (AT)
(72) Erfinder: Ebner, Walter, A-4813 Altmünster (AT)
(74) Vertreter: Hübscher, Heiner, Dipl.-Ing.

(57) **Zusammenfassung**

Zum Identifizieren von aus zugelieferten Flüssigkeitsmengen gezogenen und in Probengefäßen (5) eingefüllten Proben, welche Probengefäße (5) in einer bestimmten, durch Ordnungsgrößen festgelegten Reihenfolge in einem eine Markierung (10) mit einem maschinenlesbaren Code aufweisenden Stativ (4) od. dgl. zu einer Prüfeinheit zusammengefaßt sind, werden eine Kennung der Lieferanten und die sich aus Code und Ordnungsgröße der Prüfeinheiten ergebende Position der die entsprechenden Proben aufnehmenden Probengefäße jeweils über eine Datenverarbeitungsanlage (2) einander zugeordnet und gespeichert.

Um eine sichere und doch rationelle Identifizierung zu erreichen, wird zur Stativmarkierung jeweils ein Codeträger (10a) für ein- und auslesbare Codierungen verwendet und werden dem Codeträger eines Stativs zusätzlich zum Stativcode bei der Flüssigkeitsübernahme reihenfolgemäßig die Kennungen der den übernommenen Flüssigkeitsmengen entsprechend nacheinander in die Probengefäße eingefüllten Proben zugehörigen Lieferanten eingelesen.

## Beschreibung

Die Erfindung bezieht sich auf ein Verfahren zum Identifizieren von aus zugelieferten Flüssigkeitsmengen gezogenen und in Probengefäße eingefüllten Proben, insbesondere bei der Milchübernahme aus Lieferbehältern unterschiedlicher Lieferanten in einen Sammeltank entnommene Milchproben, welche Probengefäße in einer bestimmten, durch Ordnungsgrößen festgelegten Reihenfolge in einem eine Markierung mit einem maschinenlesbaren Code aufweisenden Stativ od. dgl. zu einer Prüfeinheit zusammengefaßt sind, wobei eine Kennung der Lieferanten und die sich aus Code und Ordnungsgröße der Prüfeinheiten ergebende Position der die entsprechenden Proben aufnehmenden Probengefäße jeweils über eine Datenverarbeitungsanlage einander zugeordnet und gespeichert werden, sowie auf eine Vorrichtung zum Durchführen dieses Verfahrens.

Um eine von einem Lieferanten übernommene Flüssikeit hinsichtlich Qualität und Eigenschaften prüfen zu können, ist es notwendig, aus dieser Flüssigkeit eine Probe zu ziehen und ihr zur Identifizierung lieferantenspezifische Daten zuzuordnen. Dies gilt für jede mit einer Prüfung verbundene Flüssigkeitsübernahme, insbesondere auch für das Übernehmen von Milch, wobei Milch aus Lieferbehältern verschiedenster Lieferanten in einen Sammeltank übernommen wird und am Übernahmeort nicht nur die in den Sammeltank eingefüllte Milchmenge festzustellen, sondern auch aus dieser Übernahmemenge jeweils eine Milchprobe zu ziehen ist. Die Proben werden nach der Übernahme in einem entsprechenden Labor untersucht und es muß möglich sein, das Untersuchungsergebnis bzw. die Probe mit dem zugehörigen Lieferanten und dessen übernommener Flüssigkeit eindeutig in Verbindung zu bringen. Dazu werden die Proben in geeignete Probengefäße gefüllt und die Zuordnung zwischen Lieferanten und Probe erfolgt über eine Identifizierung dieser Gefäße.

Wie die EP-A 0 275 072 zeigt, ist es bereits bekannt, die Probengefäße reihenweise in Stative und die Stative nebeneinander in rechteckigen Probenkasten oder hintereinander in ringförmigen Aufnahmen anzuordnen, so daß die Position des einzelnen Probengefäßes durch sein bestimmtes Stativ und seinen Platz in der Gefäßreihe des jeweiligen Stativs genau fixierbar ist. Werden die einzelnen Stative mit geeigneten Markierungen versehen und den Gefäßreihen entsprechende Ordnungsgrößen zugeordnet, können die Probengefäße positionsabhängig identifiziert und damit die in ein solches Gefäß eingefüllte Probe mit einer Kennung des jeweiligen Lieferanten verknüpft werden, was bei maschinenlesbaren Codierungen und einer entsprechenden Datenverarbeitungseinrichtung automatisch durchführbar ist. Allerdings können bei diesem Identifizierungsverfahren Fehler auftreten, wenn beispielsweise zwei Probengefäße miteinander vertauscht werden oder es zu einer Änderung der Gefäß-Reihenfolge kommt od. dgl. Um diese Fehlerquellen auszuschalten, wurde gemäß der DE-A 34 40 686 ein Identifizierungsverfahren vorgeschlagen, bei dem zusätzlich zur Bestimmung der Position eines jeden Probengefäßes in einem Probenmagazin eine Codierung jedes einzelnen Probengefäßes erfolgt, so daS einerseits über die Zuordnung von Probengefäß zu Gefäßposition und anderseits von Lieferantenkennung zu Gefäßposition auch eine eindeutige Zuordnung von Lieferantenkennung und Probengefäßen möglich ist. Dieses Verfahren bedarf aber eines beträchtlichen Aufwandes und es ist notwendig, beim Austausch der Probenmagazine, bei der Übergabe der Probenmagazine in das Labor u.s.w. jeweils die zugehörigen Koordinierungsdaten in Form von Disketten oder anderen Datenträgern dem jeweiligen Probenmagazin mitzugeben, um die Identifizierung der einzelnen Proben im Labor od. dgl. vornehmen zu können.

Aus der AT-PS 315 542 ist weiters ein Probenträger und eine Vorrichtung zum Einschreiben und Auslesen desselben bekannt, wobei auf jedem einzelnen Probenträger ein Etikett in vorbestimmter Lage befestigt ist, dem eine Information durch entsprechende Magnetisierung eines Magnetmaterials eingeschrieben werden kann, welche Information dann zur Auswertung auslesbar ist, wenn beim Einschreiben und Auslesen die lagerichtigen Positionen eingehalten werden. Dieses Indentifikationsverfahren läßt sich in klinisch-chemischen Labors einsetzen, wobei jedem Probenträger eine bestimmte Patientennummer und gegebenenfalls die Art des vorzunehmenden Analyseverfahrens mitgegeben werden, um für die Zuordnung zwischen Patient und Probe und für einen automatischen Laborbetrieb sorgen zu können. Es geht hier demnach um Einzeluntersuchungen medizinischer Proben und nicht um Serienuntersuchungen wie bei Milchproben, wozu solche Einzelgefäßkennzeichnungen viel zu aufwendig wären.

Der Erfindung liegt daher die Aufgabe zugrunde, diese Mängel zu beseitigen und ein Verfahren der eingangs geschilderten Art anzugeben, das eine sichere und vor allem auch rationelle Probenidentifizierung gewährleistet. Außerdem soll eine zweckmäßige Vorrichtung zur Durchführung dieses Verfahrens geschaffen werden.

Die Erfindung löst diese Aufgabe dadurch, daß zur Stativmarkierung jeweils ein Codeträger für ein- und auslesbare Codierungen verwendet wird und dem Codeträger eines Stativs zusätzlich zum Stativcode bei der Flüssigkeitsübernahme reihenfolgemäßig die Kennungen der den übernommenen Flüssigkeitsmengen entsprechend nacheinander in die Probengefäße eingefüllten Proben zugehörigen Lieferanten eingelesen werden. Da die Stative od. dgl. und die Probengefäße eine Prüfeinheit bilden und zumindest im Probennabmebetrieb als unteilbares Ganzes anzusehen sind, wird jeder Manipulation eines Einzelgefäßes vorgebeugt und Identifizierungsfehler durch Umsortierung der Gefäße od. dgl. sind von vornherein ausgeschlossen. Damit ist durch die Codierung des Statives und durch die Ordnungsgrößen der Gefäß-Reihenfolge die exakte Bestimmung eines beliebigen Probengefäßes möglich und durch die entsprechende Zuordnung der Lieferantenkennungen auch die exakte Probenidentifizierung gewährleistet. Wegen der dem Codeträger für die Stativmarkierungen zusätzlich einlesbaren Lieferantenkennungen werden außerdem ohne die Notwendigkeit, jedes Probengefäß selbst mit einer Codierung versehen zu müssen, dennoch jeder Prüfeinheit die erforderlichen Daten zur genauen Probenidentifizierung unmittelbar mitgegeben. Für eine spätere Handhabung der Prüfeinheiten ist keinerlei separater Datenträger od. dgl. mehr erforderlich und überall dort, wo es zu einer entsprechenden Hantierung der Prüfeinheit kommt, beispielsweise in einem Labor, ist sofort eine exakte Probenidentifizierung garantiert, was lediglich geeigneter Leseeinrichtungen bedarf. Als Codeträger lassen sich dafür grundsätzlich alle Datenträger verwenden, die das Ein- und Auslesen von Codierungen ermöglichen, wie Halbleiter-Speicherelemente, Magnetplättchen od. dgl.

Wird dem Codeträger gleichzeitig mit der Kennung eines Lieferanten auch die Ordnungsgröße des die aus der Flüssigkeitsmenge dieses Lieferanten gezogene Probe aufnehmenden Probengefäßes im jeweiligen Stativ eingelesen, kann durch die einzuspeichernde Zuordnung von Lieferantenkennung und Ordnungsgröße beim Befüllen der Probengefäße die Füllreihenfolge beliebig gewählt werden und muß sich nicht an die durch die Ordnungsgrößen an sich vorgegebene Reihenfolge halten. Abgesehen davon wird das nachträgliche Identifizieren der einzelnen Probengefäße vereinfacht und von einer zusätzlichen stativfremden Markierung für die Ordnungsgrößen unabhängig.

Das Ein- und Auslesen der Codierungen kann an und für sich auf verschiedenste Art, beispielsweise auf optischem weg oder durch Ultraschall od. dgl., vorgenommen werden, doch ist es besonders günstig, wenn dem Codeträger die Codierungen durch magnetische Kopplung über Induktionsspulen ein- und ausgelesen werden, wodurch eigene Energiequellen für den Lesevorgang unnötig sind und der Lesevorgang unempfindlich gegenüber Feuchtigkeit und Temperaturbelastungen bleibt.

Das erfindungsgemäße Probenidentifizierungsverfahren läßt sich auf im wesentlichen bekannten Vorrichtungen mit einer Probennahmeeinrichtung und einer Datenverarbeitungseinrichtung durchführen, wobei die Probennahmeeinrichtung ein Aufnahmegestell zum Einsetzen mehrerer Neben- und/oder Hintereinander angeordneter Stative od. dgl., ein von Probengefäß zu Probengefäß relativbewegbares Abfüllgerät zum Einfüllen der Proben in die Probengefäße, einen Lesekopf zum Auslesen der Stativcodierungen und einen Positionsmelder zur Angabe der Ordnungsgröße des jeweils zu befüllenden Probengefäßes aufweist und der an den Lesekopf und den Positionsmelder angeschlossenen Datenverarbeitungseinrichtung Lieferantenkennungen einlesbar sind, wenn jedes Stativ mit einem Codeträger für ein- und auslesbare Codierungen versehen ist und der Lesekopf neben dem Ausleseteil auch einen Einleseteil umfaßt, über den dem Codeträger der Stative in Abhängigkeit von der durch den Positionsmelder angegebenen Position des jeweils zu befüllenden Probengefäßes die Kennungen der den in diesen Probengefäßen abgefüllten Flüssigkeitsproben zugehörenden Lieferanten einlesbar sind. Diese Vorrichtung ermöglicht es, die bei jeder Flüssigkeitsübernahme gezogene Probe in ein bestimmtes Probengefäß einzufüllen, dessen Position aufgrund der ausgelesenen Stativcodierung und Ordnungsgröße exakt bestimmt ist, und die in der Datenverarbeitungseinrichtung eingegebenen Positionsdaten mit der zugehörenden Lieferantenkennung zu verknüpfen und eine entsprechende Codierung in den Codeträger des jeweiligen Stativs einzulesen, so daß dieser Codeträger die Informationen über die jeweiligen Proben und zugehörigen Lieferanten enthält. Die Prüfeinheiten bzw. Stative können dabei in einem Probenkasten nebeneinander gereiht angeordnet sein, sie können aber auch ringförmig hintereinander gereiht sein, denn durch die Stativmarkierung einerseits und die Ordnungsgröße anderseits ist jedes Probengefäß hinsichtlich der zugehörigen Prüfeinheit und der Position im jeweiligen Stativ genau bestimmt. Auch spielt die Anzahl der zu einer Prüfeinheit zusammengefaßten Probengefäße für deren Identifizierung keine Rolle und es sind Prüfeinheiten aus nur einem Probengefäß genauso möglich wie Prüfeinheiten mit beliebig vielen Probengefäßen.

Da die Codeträger der Stative zum Ein- und Auslesen für den Lesekopf zugänglich sein müssen, weisen die Stative vorteilhafterweise bodenseitig zugängliche Codeträger auf und der Lesekopf ist im Bodenbereich des Aufnahmegestells angeordnet, so daß durch ein Neben- oder Aneinanderreihen der Stative keine gegenseitige Beeinträchtigung dieser Zugänglichkeit befürchtet zu werden braucht.

Ist der Lesekopf ortsfest im Aufnahmegestell eingesetzt und weist in Zahl und Anordnung an die aufnehmbaren Stative und deren Codeträger angepaßte Leseeinheiten auf, wird das Ein- und Auslesen der Codierungen erleichtert, da eine exakte örtliche Zuordnung zwischen Leseeinheit und Codeträger möglich ist und es zu keinen bewegungs- oder lagebedingten Störungen der Lesevorgänge kommt.

Umfaßt der Lesekopf als Aus- und Einleseteile Induktionsspulen, kommt es zu einer einfachen, aufwandsarmen und funktionsgerechten Bauweise, die ein datensicheres Lesen mittels magnetischen Kopplung gewährleistet.

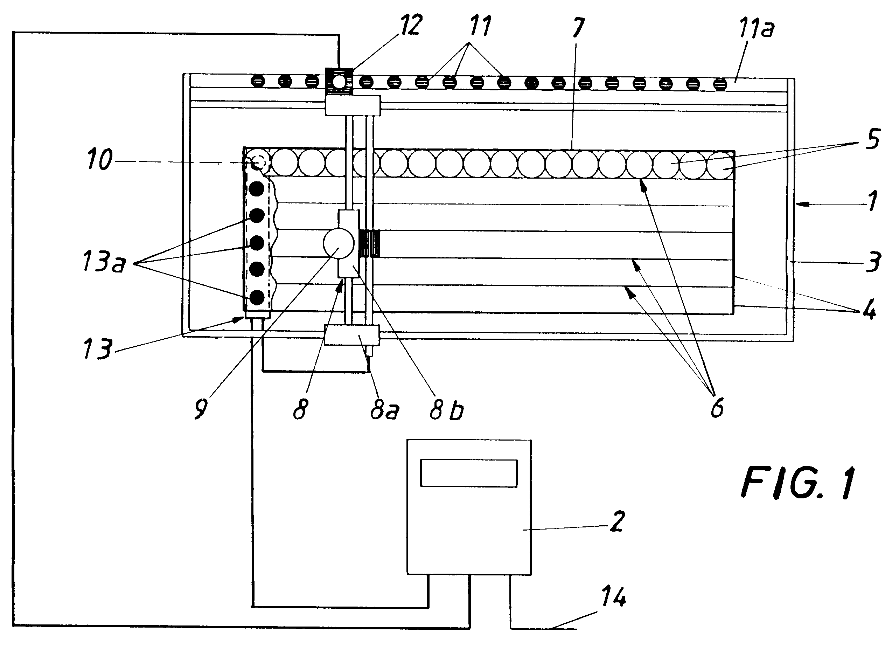

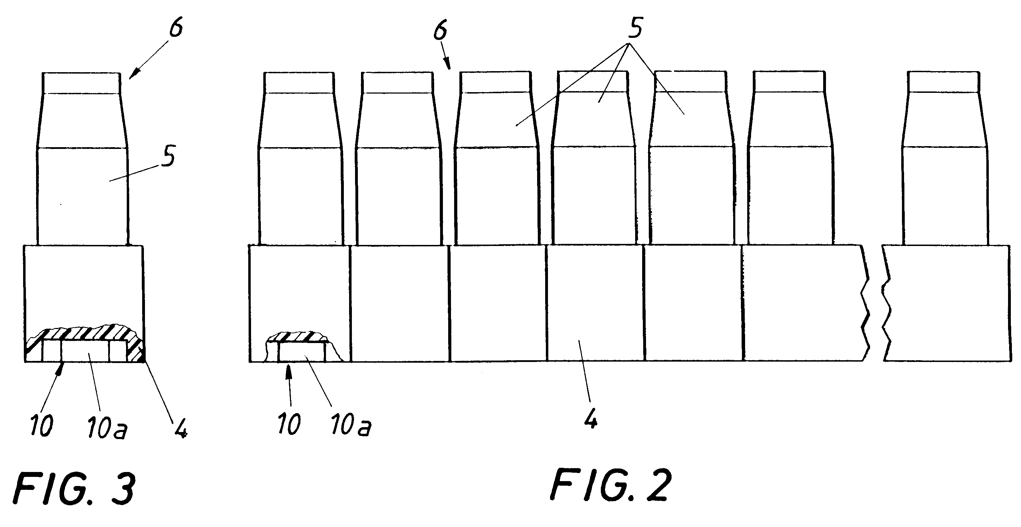

In der Zeichnung ist der Erfindungsgegenstand rein schematisch veranschaulicht, und zwar zeigen Fig. 1 einen Teil einer erfindungsgemäßen Vorrichtung zur Probenidentifizierung in Draufsicht und Fig. 2 und 3 eine Prüfeinheit für diese Vorrichtung in Seiten- und Stirnansicht.

Um eindeutig eine aus zugelieferten Flüssigkeitsmengen, beispielsweise Milch, gezogene und in ein Probengefäß eingefüllte Probe identifizieren zu können, sind eine Probennahmevorrichtung 1 und eine Datenverarbeitungseinrichtung 2 vorgesehen. Die Probennahmeeinrichtung 1 weist ein Aufnahmegestell 3 zum Einsetzen mehrerer nebeneinander angeordneter Stative 4 auf, in welchen Stativen 4 jeweils eine Reihe von Probengefäßen 5 sitzen und zu einer Prüfeinheit 6 zusammengefaßt sind. Ein Probenkasten 7 nimmt die in die Probennahmeeinrichtung 1 einzusetzenden Prüfeinheiten 6 nebeneinander auf und erleichtert so deren Handhabung beim Einsetzen in oder Herausnehmen aus dem Aufnahmegestell 3. Auf einem Kreuzschlitten 8, der sich aus einem in Längsrichtung der Stative 4 verfahrbaren Längsschlitten 8a und einem quer dazu verfahrbaren Querschlitten 8b zusammensetzt, ist eine Abfülleinrichtung 9 von Probengefäß zu Probengefäß bewegbar, mit der Milchproben od. dgl. in die einzelnen Probengefäße 5 eingefüllt werden können.

Zur Identifizierung des jeweiligen Probengefäßes 5 ist jedes Stativ 4 mit einer Stativmarkierung 10 versehen und die Reihenfolge der einzelnen Gefäße 5 in jedem Stativ 4 wird durch Ordnungsgrößen in Form von Reihenmarkierungen 11 auf einem gestellfesten Markierungsträger 11a festgelegt. Für das Erkennen dieser Markierungen 10, 11 gibt es einen Positionsmelder 12 zum Erfassen der Reihenmarkierungen 11 und einen Lesekopf 13 zum Auslesen der Stativmarkierungen 10, wobei Positionsmelder 12 und Lesekopf 13 an die Datenverarbeitungseinrichtung 2 angeschlossen sind. An diese Datenverarbeitungseinrichtung ist in nicht weiter dargestellter Weise über eine Informationsleitung 14 eine Kennung der jeweiligen Lieferanten einlesbar, wobei diese Kenndateneinlesung vorzugsweise beim Übernehmen der Milch aus den Lieferbehältern in den Sammeltank erfolgt. Damit ist es auch möglich, diese Lieferantenkennungen mit der jeweiligen Position des Probengefäßes 5 zu verbinden, in das die aus der Flüssigkeit dieses Lieferanten gezogene Probe gerade eingefüllt wird.

Die Stativmarkierungen 10 bestehen nun aus einem Codeträger 10a für ein- und auslesbare Codierungen, so daß aus diesem Codeträger 10a nicht nur die jeweilige Stativcodierung ausgelesen werden kann, sondern es auch möglich ist, in den Codeträger die den einzelnen Probengefäßen des jeweiligen Statives reihenfolgegemäß zugeordneten Lieferantenkennungen einzulesen, wodurch die zur Identifizierung der Proben erforderlichen Daten auf den Prüfeinheiten 6 selbst eingespeichert sind. Dazu weist der Lesekopf 13 zusätzlich zu seinen Ausleseteilen auch Einleseteile auf, welche Teile vorzugsweise als gemeinsame Leseeinheiten 13a mit Induktionsspulen zur induktiven Kopplung vorgesehen sind. Diese Leseköpfe 13 und die entsprechenden Codeträger 10a ermöglichen es, bedarfsweise Codierungen aus der Stativmarkierung 10 auszulesen und sie dann ergänzt durch Lieferantenkennungen, Zeit- und Mengenangaben od. dgl. wieder einzulesen.

Um ein ungestörtes Zusammenwirken zwischen Lesekopf einerseits und Codeträger anderseits sicherzustellen, ist der Lesekopf 13 im Bodenbereich des Aufnahmegestells 3 eingesetzt und für jede Prüfeinheit 5, die im Aufnahmegestell 3 Platz findet, mit einer eigenen Leseeinheit 13a ausgestattet, und werden die Codeträger 10 der Prüfeinheiten 6 bzw. der Stative 3 bodenseitig zugänglich angeordnet. Sobald daher der Probenkasten 7 mit den Prüfeinheiten 6 in das Aufnahmegestell 3 eingesetzt ist, liegen sich zwangsweise die Leseeinheiten 13a des Lesekopfes 13 und die Codeträger 10a im erforderlichen Abstand gegenüber und die Lesevorgänge sind einwandfrei durchführbar.

Ist die Speicherkapazität der Codeträger 10a groß genug, lassen sich in diese Stativmarkierung 10 nicht nur die Lieferantenkennungen und die Stativcodierung an sich, sondern auch die Reihenmarkierungen 11 für die Ordnungsgrößen einlesen, was die direkte Zuordnung von Lieferantenkennung und Ordnungsgröße mit sich bringt und die Probenidentifizierung weiter vereinfacht.

Da die Probengefäße 5 und die sie aufnehmenden Stative 4 für den Probennahmenbetrieb eine unteilbare Prüfeinheit bilden, können die einzelnen Probengefäße 5 innerhalb der Prüfeinheiten 6 nicht vertauscht werden und durch die Codierung der Prüfeinheiten hinsichtlich Stativbezeichnung, Lieferantenkennungen und gegebenenfalls der Ordnungsgrößen für die Gefäßreihenfolge ist eine sichere und eindeutige Identifizierung der einzelnen Probengefäße 5 und damit der in diesen Probengefäßen eingefüllten Flüssigkeitsproben gewährleistet.

## Patentansprüche

1. Verfahren zum Identifizieren von aus zugelieferten Flüssigkeitsmengen gezogenen und in Probengefäße eingefüllten Proben, insbesondere bei der Milchübernahme aus Lieferbehältern unterschiedlicher Lieferanten in einen Sammeltank entnommene Milchproben, welche Probengefäße in einer bestimmten, durch Ordnungsgrößen festgelegten Reihenfolge in einem eine Markierung mit einem maschinenlesbaren Code aufweisenden Stativ od. dgl. zu einer Prüfeinheit zusammengefaßt sind, wobei eine Kennung der Lieferanten und die sich aus Code und Ordnungsgröße der Prüfeinheiten ergebende Position der die entsprechenden Proben aufnehmenden Probengefäße jeweils über eine Datenverarbeitungsanlage einander zugeordnet und gespeichert werden, dadurch gekennzeichnet, daß zur Stativmarkierung jeweils ein Codeträger für einund auslesbsare Codierungen verwendet wird und dem Codeträger eines Stativs zusätzlich zum Stativcode bei der Flüssigkeitsübernahme reihenfolgemäßig die Kennungen der den übernommenen Flüssigkeitsmengen entsprechend nacheinander in die Probengefäße eingefüllten Proben zugehörigen Lieferanten eingelesen werden.

2. Verfahren nach Anspruch 1, dadurch gekennzeichnet, daß dem Codeträger gleichzeitig mit der Kennung eines Lieferanten auch die Ordnungsgröße des die aus der Flüssigkeitsmenge dieses Lieferanten gezogene Probe aufnehmenden Probengefäßes im jeweiligen Stativ eingelesen wird.

3. Verfahren nach Anspruch 1 oder 2, dadurch gekennzeichnet, daß dem Codeträger die Codierungen durch magnetische Kopplung über Induktionsspulen ein- und ausgelesen werden.

4. Vorrichtung zur Durchführung des Verfahrens nach einem der Ansprüche 1 bis 3, mit einer Probennahmeeinrichtung, die ein Aufnahmegestell zum Einsetzen mehrerer neben- und/oder hintereinander angeordneter Stative od. dgl., ein von Probengefäß zu Probengefäß relativbewegbares Abfüllgerät zum Einfüllen der Proben in die Probengefäße, einen Lesekopf zum Auslesen der Stativcodierungen und einen Positionsmelder zur Angabe der Ordnungsgröße des jeweils zu befüllenden Probengefäßes aufweist, und mit einer Datenverarbeitungseinrichtung, welcher an den Lesekopf und den Positionsmelder angeschlossenen Datenverarbeitungseinrichtung Lieferantenkennungen einlesbar sind, dadurch gekennzeichnet, daß jedes Stativ (4) mit einem Codeträger (10a) für ein- und auslesbare Codierungen versehen ist und der Lesekopf (13) neben dem Ausleseteil auch einen Einleseteil umfaßt, über den dem Codeträger (10a) der Stative (4) in Abhängigkeit von der durch den Positionsmelder (12) angegebenen Position des jeweils zu befüllenden Probengefäßes (5) die Kennungen der den in diesen Probengefäßen abgefüllten Flüssigkeitsproben zugehörenden Lieferanten einlesbar sind.

5. Vorrichtung nach Anspruch 4, dadurch gekennzeichnet, daß die Stative (4) bodenseitig zugängliche Codeträger (10a) aufweisen und der Lesekopf (13) im Bodenbereich des Aufnahmegestells (3) angeordnet ist.

6. Vorrichtung nach Anspruch 4 oder 5, dadurch gekennzeichnet, daß der Lesekopf (13) ortsfest im Aufnahmegestell (3) eingesetzt ist und in Zahl und Anordnung an die aufnehmbaren Stative (4) und deren Codeträgern (10a) angepaßte Leseeinheiten (13a) aufweist.

7. Vorrichtung nach einem der Ansprüche 4 bis 6, dadurch gekennzeichnet, daß der Lesekopf (13) als Aus- und Einleseteile Induktionsspulen umfaßt.
